# EUROPEAN PATENT SPECIFICATION

(11) **EP 2 611 763 B1**
(45) Date of publication and mention of the grant of the patent: **18.06.2014**
(21) Application number: 10765494.9
(22) Date of filing: 03.09.2010
(51) Int. Cl.: C07C 46/04, C07C 50/10

(54) **PROCESS FOR THE INDUSTRIAL PRODUCTION OF 2-METHYL-1,4-NAPHTHOQUINONE**
VERFAHREN ZUR INDUSTRIELLEN HERSTELLUNG VON 2 -METHYL - 1, 4 - NAPHTHOCHINON
PROCÉDÉ POUR LA PRODUCTION INDUSTRIELLE DE LA 2-MÉTHYL-1,4-NAPHTOQUINONE

(43) Date of publication of application: 10.07.2013
(73) Proprietor: Tubitak, 06100 Ankara (TR)
(72) Inventor: YALCIN, Seher, 41470 Kocaeli (TR); CANDEMIR, Mustafa, 41470 Kocaeli (TR); KORLU, Zekai, 41470 Kocaeli (TR); BEKIR, Nevin, 41470 Kocaeli (TR); OZKARAARSLAN, Murat, 41470 Kocaeli (TR); KAYDI, Izzet, 41470 Kocaeli (TR)
(74) Representative: Yavuzcan, Alev
(86) International application number: PCT/IB2010/053967
(87) International publication number: WO 2012/028905

(56) References cited:
- EP-A1- 0 704 421
- WO-A1-02/079133
- WO-A2-2005/123644
- CN-A- 101 575 276
- ARNOLD R T ET AL: "QUINONES BY THE PEROXIDE OXIDATION OF AROMATIC COMPOUNDS", JOURNAL OF ORGANIC CHEMISTRY, AMERICAN CHEMICAL SOCIETY, EASTON.; US, vol. 5, 1 January 1940 (1940-01-01), pages 250-252, XP001041459, ISSN: 0022-3263, DOI: DOI:10.1021/JO01209A006
- DATABASE WPI Week 199421 Thomson Scientific, London, GB; AN 1994-175246 XP002635501, & SU 1 803 401 A1 (KHARK POLY) 23 March 1993 (1993-03-23)
- DATABASE WPI Week 197824 Thomson Scientific, London, GB; AN 1978-43081A XP002635502, & JP 53 050147 A (NIPPON JORYU KOGYO KK) 8 May 1978 (1978-05-08)
- NARENDER N ET AL: "Liquid phase oxidation reactions over MoMCM-41 and WMCM- 41molecular sieves", FROM ZEOLITES TO POROUS MOF MATERIALS : THE 40TH ANNIVERSARY OF INTERNATIONAL ZEOLITE CONFERENCE; PROCEEDINGS OF THE 15TH INTERNATIONAL ZEOLITE CONFERENCE, BEIJING, P.R. CHINA, 12-17TH AUGUST 2007; [STUDIES IN SURFACE SCIENCE AND CATALYSIS], AMSTERDA, vol. 170 B, 12 August 2007 (2007-08-12), pages 1877-1883, XP008136245, DOI: DOI:10.1016/S0167-2991(07)81073-1 ISBN: 978-0-444-53186-5 [retrieved on 2007-10-18]

## Description

### TECHNICAL FIELD

The present invention relates to chemistry, pharmacy and vitamins.

### BACKGROUND ART

The industrial production of 2-methyl-1,4-naphthoqulnone (menadione, vitamine K3) is a challenging problem because of its pharmacological activities. It is used as blood coagulating agent and as animal feed supplement. A lot of methods have been developed to oxidize 2-methylnaphthalene for the synthesis of 2-methyl-1,4-naphthoquinone.

In a conventional process, 2-methylnapthalene is oxidized with stoichiometric amount of chromium (VI) compounds in sulfuric acid (Sheldon, Top. Curr. Chem., 164, 1993, 21). However, production of chromium salts are side products (18 kg of salt per kg of 2-methyl-1,4-naphthoquinone) and necessary treatment of this waste made it difficult to produce 2-methyl-1,4-naphthoquinone by this process in these days.

Alternative procedures using Mn(III) (Periasamy et al., Tetrahedron Lett. 4, 1978, 4561) and Ce (IV) (Kreh et al., J. Org. Chem. 54, 1989, 1526) have been proposed Although, Ce(IV) can be regenerated by electrolysis, the stoichiometric use of transition metal oxidants for industry is undesirable from economic and environmental view.

On the other hand, Iron, palladium, rhenium, ruthenium, porphyrin, phthalocynanin and zeolite complexes are used as catalyst where hydrogen peroxide, percarboxylic acids or dioxygen are applied as the oxygen source. Fe(III) activated oxidation of 2-methylnaphthalene in the presence of hydrogen peroxide results in low yield of 2-methyl-1,4-naphthoquinone with long reaction time (Kowalski et al., Catalysis Comm 4, 2003, 603). Oxidation of 2-methylnaphthalene with Re (IV, VI and VII) and salts were studied in the presence of highly concentrated hydrogen peroxide. In these cases, low to moderate yields and selectivity are obtained (Herrmann et al., J. Mol. Cat. A: Chem. 138, 1999, 115; Herrmann et al., US Patent 5710292). Another catalysis for the oxidation of 2-methylnaphthalene is Ru (II) with phase transfer catalysts which results in moderate conversion and regioselectivity (Shi et al., J. Mol. Cat. A: Chem. 270, 2007, 68). Water-soluble metalloporphyrins are another catalysts for the oxidation of 2-methylnaphthalene where potassium monopersulfate as primary oxidant (Meunier et al., J. Org. Chem. 62, 1997, 673). Fe and Mn porphyrines catalyzed oxidation of 2-methylnaphthalene results in high yield with low conversion and regioselectivity. Pd is used with polystyrene resin is used in another attempt (Yamaguchi et al., Chem.Lett. 1985, 827) Because of using great amount of resin relative to 2-methylnaphthalene, loosing catalytic activity after 5 to 6 use and contamination of resin bring very high cost to this process. In addition, palladium acetate oxidizes 2-methylnaphthalene in the presence of hydrogen peroxide (Yoichi et al., US Patent, US5637741). This procedure results in moderate yield. When zeolites are used for the synthesis of 2-methyl-1,4-naphthoquinone with hydrogen peroxide as oxidant, reaction yield is moderate (Anunziata et al., J. Mol. Catal. A:Chem. 149, 1999, 255). In brief, economical concerns, hazard potential, low regioselectivity and yield and make these procedures difficult to apply to the industry. Alternatively, for the oxidation of 2-methylnaphthalene, mineral acids (e.g. H2SO4) are used in the presence of hydrogen peroxide and good yield and selectivity of the product is obtained (Thiel et al., PCT Patent WO2005123644).

These oxidation reactions of 2-methylnaphthalene in the presence of transition metal of mineral acid catalysts brings drawbacks in the environmental and economical point of view. Corrosion of reactors and vessels, metal and acid wastes, problems with the reuse of the catalysts, low regioselectivity and yield.

Oxidation of 2-methylnaphthalene with hydrogen peroxide without any transition metal or mineral acid catalyst was studied (Sankarasubbier et al., PCT Patent WO2002079133). In this case, molar ratios of 2-methylnaphthalene to hydrogen peroxide are in the range of 1:2 to 1:10 in the presence of acetic acid wherein the concentration of acetic acid Is 5-17 N. This process for the oxidation of 2-2-methylnaphthalene results in moderate to high yield and selectivity of 2-methyl-1,4-naphthoquinone. For the high yield and regioselectivity, molar ratios of 2-methylnaphthalene to hydrogen peroxide must be 1.10 in the presence of 17N acetic acid with 100°C reaction temperature and 3 hours of reaction time.

### DISCLOSURE OF THE INVENTION

For the oxidation of 2-methylnaphthalene to produce 2-methyl-1,4-naphthoquinone, in the prior art processes, metal oxidizing agents (e.g. chromium salts) were used with stoichiometric amount or transition metal catalysts (e.g. ruthenium, cerium) were used with hydrogen peroxide or molecular oxygen. In addition, mineral acids (e.g. H₂SO₄) were used as catalyst in the presence of acetic acid when hydrogen peroxide was used as oxidizing reagent. In the present invention, for the oxidation process of 2-methylnphthalene, hydrogen peroxide and glacial acetic acid were used. The present invention does not include any metal oxidizing agents, transition metal catalysts or mineral acid.

Moreover, in the prior art process, 2-methylnaphthalene was oxidized with hydrogen peroxide In the presence of acetic acid (WO2002079133). The prior art process and the present invention used different molar ratios of hydrogen peroxide and acetic acid at different time periods of the reactions.

In the prior art process, 2-methylnaphthalene was oxidized with hydrogen peroxide wherein molar ratios of 2-methylnaphthalene to hydrogen peroxide were in the range of 1:2 to 1:12 in the presence of acetic acid wherein concentration of acetic acid was 5-17 N and reaction duration was 1 to 3 hours with 60-100 °C temperature range resulting In moderate to high yield and selectivity of 2-methyl-1,4-naphthoquinone. In the present invention, the molar ratios of 2-methylnaphthalene to hydrogen peroxide is in the range of 1:15 to 1:30 in the presence of glacial acetic acid wherein concentration of acetic acid is 17,4 N and reaction duration is 4 to 6 hours in 60-90 °C temperature range. Molar ratio of 2- methylnaphthalene to hydrogen peroxide and concentration of acetic acid and reaction duration are given In Table 1 for the prior art process (WO2002079133) and the present invention.

**Table 1: Molar ratio of 2-methylnaphthalene to hydrogen peroxide and concentration of acetic acid and reaction duration for the prior art process (WO2002079133) and the present invention**

| | Molar ratio of 2-methylnaphthale ne to hydrogen peroxide (30 %) | Concentration of acetic acid (N) | Reaction time (hours) |
|---|---|---|---|
| W02002079133 | 1:2 to 1:12 | 5 to 17 | 1 to 3 |
| The present invention | 1:15 to 1:30 | 17,4 | 4 to 6 |

In addition, for the 100 % yield and selectivity, molar ratio of 2-methylnaphthalene to hydrogen peroxide was 1:10 and molar ratio of 2-methylnaphthalene to 17 N acetic acid was 1:50 at 100 °C for 3 hours reaction In the prior art process (WO2002079133). In the present invention, both the molar ratio of 2-methylnaphthalene to hydrogen peroxide and the molar ratio of 2-methylnaphthalene to glacial acetic acid (17,4 N) are 1:20 with the 4 to 6 reaction time. Molar ratios of 2-methylnaphthalene to hydrogen peroxide and acetic acid are given in Table 2 for the 100 % yield and selectivity of 2-methyl-1,4-naphthoquinone.

**Table 2: Molar ratios of 2-methylnaphthalene to hydrogen peroxide and acetic acid with reaction conditions in the prior art process (WO2002079133) and the present invention for the 100 % yield selectivity.**

| | Molar ratio of 2-methylnaphthalene to hydrogen peroxide | Molar ratio of 2-methylnaphthalene to acetic acid | Reaction time (hours) | Reaction temperature (°C) | Yield and selectivity of 2-methyl-1,4-naphthoquinone (%) |
|---|---|---|---|---|---|
| WO200207913 3 | 1:10 | 1:50 | 3 | 100 | 100 |
| The present invention | 1:20 | 1:20 | 5 | 75 | 100 |

The hydrogen peroxide must be added to the reaction mixture after heating to 60-90 °C. Low yield of 2-methyl-1,4-naphthoquinone is obtained if it is added in the beginning of the oxidation reaction. Furthermore, in the prior art process it was stated that the hydrogen peroxide was added slowly to the reaction mixture. In the present invention, addition of the hydrogen peroxide to the reaction mixture is quick (in 3 minutes); in case of the slow addition (in 10 minutes or more) reduced yield of 2-methyl-1,4-naphthoquinone is obtained.

In brief, the present invention does not include any metal oxidizing agents, transition metal catalysts or mineral acid for the oxidation of 2-methylnaphthalene to produce 2-methyl-1,4-naphthoquinone. The present invention provides an economical and ecofriendly process for the 100 % yield and regloselectivity of target product 2-methyl-1,4-naphthoquinone by using different molar ratios of 2-methylnaphthalene to hydrogen peroxide and acetic acid which is glacial acetic acid (conc. 17,4) and reaction time than the prior art processes.

### DETAILED DESCRIPTION OF THE INVENTION

The present invention provides a process for oxidation of 2-methylnaphthalene to 2-melhyl-1,4-naphthoquinone. Starting material 2-methylnaphthatene is industrially produced and commercially available. 30% Aqueous solution of fresh hydrogen peroxide is used as oxidizing agent and must be added to the reaction mixture after heating. Low yield of 2-methyl-1,4-naphthoquinone is obtained if it is added in the beginning of the oxidation reaction. Furthermore, addition of the hydrogen peroxide to the reaction mixture is quick (in 3 minutes); in case of the slow addition (in 10 minutes or more) reduced yield of 2-methyl-1,4-naphthoquinone is obtained. Glacial acetic acid is used as solvent In the present invention. The reactions are analyzed by using Bruker Avance-500 NMR Spectrometer, Agilent 6890N GC and Agilent 5973 GC-MS with HP-5MSI column coupled with flame ionization detector The present invention studied in the absence of any transition metal or mineral acid catalyst.

### EXAMPLE 1

A solution of 2-methylnaphthalene (5 gr, 35.2 mmol) in glacial acetic (40,5 ml, 0.703 mol) acid is heated to 75 °C under stirring. After reaching to 75 °C, hydrogen peroxide (30%) is added in 3 minutes and the resulting mixture is stirred for further 5 hours at 75 °C. The reactions are analyzed using GC, GC-MS and NMR. Different molar ratios of 2-methylnaphthalene and hydrogen peroxide (30%) are studied. The results of the conversion and selectivity are given in Table 3.

**Table 3: Conversion and selectivities changing 30 % aqueous hydrogen peroxide molar ratio**

| Amount of hydrogen peroxide (30 %) | Molar ratio of 2-methylnaphthalene to hydrogen peroxide | Conversion of 2-methylnaphthalene (%) | Selectivity of 2-methyl-1,4-naphthoquinone |
|---|---|---|---|
| 54 ml (0.527 mol) | 1:15 | 93 | 93 |
| 72 ml (0.703 mol) | 1:20 | 100 | 100 |
| 108 ml (1.054 mol) | 1:30 | 100 | 100 |

### EXAMPLE 2

A solution of 2-methylnaphthalene (5 gr, 35.2 mmol) in glacial acetic (40,5 ml, 0.703 mol) is heated to 75 °C under stirring After reaching to 75 °C, 30% aqueous hydrogen peroxide (72 ml, 0.703 mol) is added in 3 minutes and the resulting mixture is continued stirring at 75 °C The reaction is studied at different reaction durations from 4 to 6 hours. The reactions are analyzed by GC, GC-MS and NMR. The results of the conversion and selectivity are given in Table 4.

**Table 4: Conversion and selectivities at 4-6 reaction times duration.**

| Reaction durations (hrs) | Conversion of 2-methylnaphthalene (%) | Selectivity of 2-methyl-1,4-naphthoquinone |
|---|---|---|
| 4 | 89 | 100 |
| 5 | 100 | 100 |
| 6 | 100 | 100 |

### EXAMPLE 3

A solution of 2-methylnaphthalene (5 gr, 35.2 mmol) in glacial acetic acid is heated to 75 °C under stirring. After reaching to 75 °C, 30 % aqueous hydrogen peroxide (72 ml, 0.703 mol) is added in 3 minutes and the resulting mixture is stirred for further 5 hours at 75 °C. The reactions are analyzed by GC, GC-MS and NMR. The results of the conversion and selectivity are given in Table 5.

**Table 5: Conversion and selectivities changing glacial acetic acid molar ratio**

| Amount of Glacial Acetic Acid added | Molar ratio of 2-methylnaphthalene to acetic acid | Conversion of 2-methylnaphthalene (%) | Selectivity of 2-methyl-1,4-naphthoquinone |
|---|---|---|---|
| 30.5 ml (0.53 mol) | 1:15 | 89 | 89 |
| 40,5 ml (0.703 mol) | 1:20 | 100 | 100 |
| 60.5 ml (1.054 mol) | 1:30 | 100 | 100 |

2-Methylnaphthalene was oxidized to 2-methyl-1,4-naphthoquinone with hydrogen peroxide in glacial acetic acid. For this oxidation process neither mineral nor transition metal catalysts are used which means resulted in no waste and environmentally convenient process. Additionally, it is very economical process because of the high conversion and selectivity. In this reaction reactors and vessels are prevented of from corrosion which brings the additional economy to the oxidation of 2-Methylnaphthalene.

The prior art process (WO2002079133) have shown the oxidation of 2-methyl-1,4-naphthoquinone with hydrogen peroxide in acetic acid. The present invention brings much more economical process to the industrial production of 2-methyl-1,4-naphthoquinone by using half amount reactants with less amount of energy for heating lower degree for obtaining same results. As a result, the present invention lowers the cost than in prior art processes.

### TECHNICAL PROBLEM

The industrial production of 2-methyl-1,4-naphthoquinone (menadione, vitamine K₃) is a challenging problem because of its pharmacological activities. A lot of methods have been developed to oxidize 2-methylnaphthalene for the synthesis of 2-methyl-1,4-naphthoquinone. These oxidation reactions of 2-methylnaphthalene in the presence of transition metal or mineral acid catalysts bring drawbacks in the environmental and economical point of view: Corrosion of reactors and vessels, metal and acid wastes, problems with the reuse of the catalysts, low regloselectivity and yield. In the present invention, neither transition metal catalysts nor mineral acids are used for oxidation of 2-methylnaphthalene for the synthesis of 2-methyl-1,4-naphthoquinone. The present invention provides an economical and environmentally friendly process resulting in high yield of 2-methyl-1,4-naphthoquinone without waste, corrosion of reactors.

Besides these processes, oxidation of 2-methylnaphthalene with hydrogen peroxide without any transition metal or mineral acid catalyst was studied in prior art process (Sankarasubbier et al., PCT Patent WO2002079133). Although this prior art process resulted in moderate to high yield and selectivity, it is not economical in order to apply to industrial production For example, according to the previous art process in order to oxidize 10 gr of 2-methylnaphthalene to synthesize 2-methyl-1,4-naphthoquinone in 100 % yield and selectivity, 72 ml of 30% aqueous hydrogen peroxide and 200 ml of 17 N acetic acid were needed. Total amount of oxidizing agent and solvent were 272 ml and must be heated to 100 °C. On the other hand, in the present invention for the oxidation of same amount of 2-methylnaphthalene with 100 % yield and selectivity, total amount of oxidizing agent and solvent are 225 ml and heated to 75 °C (Table 6).

In brief, the present invention provides a much more economical process than prior art process (WO2002079133) by using reduced amount of reactants and temperature: Reduced cost, reduced mixing and heating energy. Comparison of WO2002079133 and the present invention by means of amount of reactants and temperature is given in Table 6.

**Table 6: Amount of reactants and temperature in WO2002079133 and in the present invention.**

| | Amount of 30% aqueous hydrogen peroxide added (ml) | Amount of acetic acid added (ml) | Reaction temperature (°C) | **Total amount of oxidizing agent and solvent (ml)** | Yield and selectivity of 2-methyl-1,4-naphthoquinone (%) |
|---|---|---|---|---|---|
| WO20020 79133 | 72 | 200 | 100 | **272** | 100 |
| Present invention | 144 | 81 | 75 | **225** | 100 |

### TECHNICAL SOLUTION

For the oxidation of 2-methylnaphthalene to produce 2-methyl-1,4-naphthoquinone, in the prior art processes, metal oxidizing agents (e.g. chromium salts) were used with stoichiometric amount or transition metal catalysts (e.g. ruthenium, cerium) were used with hydrogen peroxide or molecular oxygen. Also, mineral acids (e.g. H₂SO₄) were used as catalyst in the presence of acetic acid when hydrogen peroxide was used as oxidizing reagent. In the present invention, for the oxidation process of 2-methylnphthalene, hydrogen peroxide and glacial acetic acid were used. The first difference between the present invention and the prior art processes is that the present invention does not include any metal oxidizing agents, transition metal catalysts or mineral acid.

Moreover, in the prior art process, 2-methylnaphthalene was oxidized with hydrogen peroxide in the presence of acetic acid (WO2002079133). The prior art process and the present invention used different molar ratios of hydrogen peroxide and acetic acid at different time periods of the reactions.

In the prior art process, 2-methylnaphthalene was oxidized with hydrogen peroxide wherein molar ratios of 2-methylnaphthalene to hydrogen peroxide were in the range of 1:2 to 1:12 in the presence of acetic acid wherein concentration of acetic acid was 5-17 N and reaction duration was 1 to 3 hours with 60-100 °C temperature range resulting In moderate to high yield and selectivity of 2-methyl-1,4-naphthoquinone. In the present invention, the molar ratios of 2-methylnaphthalene to hydrogen peroxide is in the range of 1:15 to 1:30 in the presence of glacial acetic acid wherein concentration of acetic acid is 17,4 N and reaction duration is 4 to 6 hours in 60-90 °C temperature range. Molar ratio of 2- methylnaphthalene to hydrogen peroxide and concentration of acetic acid and reaction duration are given in Table 7 for the prior art process (WO2002079133) and the present invention.

**Table 7: Molar ratio of 2-methylnaphthalene to hydrogen peroxide and concentration of acetic acid and reaction duration for the prior art process (WO2002079133) and the present invention**

| | Molar ratio of 2-methylnaphthalene to hydrogen peroxide (30 %) | Concentration of acetic acid (N) | Reaction time (hours) |
|---|---|---|---|
| WO2002079133 | 1:2 to 1:12 | 5 to 17 | 1 to 3 |
| The present invention | 1:15 to 1:30 | 17,4 | 4 to 6 |

In addition, for the 100 % yield and selectivity, molar ratio of 2-methylnaphthalene to hydrogen peroxide was 1:10 and molar ratio of 2-methylnaphthalene to 17 N acetic acid was 1:50 at 100 °C for 3 hours reaction in the prior art process (WO2002079133). In the present invention, both the molar ratio of 2-methylnaphthalene to hydrogen peroxide and the molar ratio of 2-methylnaphthalene to glacial acetic acid (17,4 N) are 1:20 with the 4 to 6 reaction time. Molar ratios of 2-methylnaphthalene to hydrogen peroxide and acetic acid are given in Table 8 for the 100 % yield and selectivity of 2-methyl-1,4-naphthoquinone.

**Table 8: Molar ratios of 2-methylnaphthalene to hydrogen peroxide and acetic acid with reaction conditions in the prior art process (WO2002079133) and the present invention for the 100 % yield selectivity.**

| | Molar ratio of 2-methylnaphthalene to hydrogen peroxide | Molar ratio of 2-methylnaphthalene to acetic acid | Reaction time (hours) | Reaction temperature (°C) | Yield and selectivity of 2-methyl-1,4-naphthoquinone (%) |
|---|---|---|---|---|---|
| WO2002079 133 | 1:10 | 1:50 | 3 | 100 | 100 |
| The present invention | 1:20 | 1:20 | 5 | 75 | 100 |

The hydrogen peroxide must be added to the reaction mixture after heating to 60-90 °C. Low yield of 2-methyl-1,4-naphthoquinone is obtained if it is added in the beginning of the oxidation reaction. Furthermore, in the prior art process it was stated that the hydrogen peroxide was added slowly to the reaction mixture. In the present invention, addition of the hydrogen peroxide to the reaction mixture is quick (in 3 minutes); in case of the slow addition (in 10 minutes or more) reduced yield of 2-methyl-1,4-naphthoquinone is obtained.

In brief, the present invention does not include any metal oxidizing agents, transition metal catalysts or mineral acid for the oxidation of 2-methylnaphthalene to produce 2-methyl-1,4-naphthoquinone. The present invention provides an economical and ecofriendly process for the 100 % yield and regioselectivity of target product 2-methyl-1,4-naphthoquinone by using different molar ratios of 2-methylnaphthalene to hydrogen peroxide and acetic acid which is glacial acetic acid (conc. 17,4) and reaction time than the prior art processes.

### ADVANTAGEOUS EFFECTS

For the oxidation of 2-methylnaphthalene to produce 2-methyl-1,4-naphthoquinone, in the prior art processes, metal oxidizing agents (e.g. chromium salts) were used with stoichiometric amount or transition metal catalysts (e.g. ruthenium, cerium) were used with hydrogen peroxide or molecular oxygen. Also, mineral acids (e.g. H₂SO₄) were used as catalyst in the presence of acetic acid when hydrogen peroxide was used as oxidizing reagent. In the present invention, for the oxidation process of 2-methylnphthalene, hydrogen peroxide and glacial acetic acid were used and neither mineral nor transition metal catalysts are used which means resulted in no waste and environmentally convenient process. Additionally, it is very economical process because of the high conversion and selectivity. In this reaction reactors and vessels are prevented of from corrosion which brings the additional economy to the oxidation of 2-methylnaphthalene.

Moreover, in the prior art process, 2-methylnaphthalene was oxidized with hydrogen peroxide in the presence of acetic acid (WO2002079133). The prior art process and the present invention used different molar ratios of hydrogen peroxide and acetic acid at different time periods of reaction. Although this prior art process resulted in moderate to high yield and selectivity, it is not economical in order to apply to industrial production.

The present invention brings much more economical process to the industrial production of 2-methyl-1,4-naphthoquinone by using half amount reactants with less amount of energy for heating lower degree for obtaining same results. For example, according to the previous art process in order to oxidize 10 gr of 2-methylnaphthalene to synthesize 2-methyl-1,4-naphthoquinone in 100 % yield and selectivity, 72 ml of 30% aqueous hydrogen peroxide and 200 ml of 17 N acetic acid were needed. Total amount of oxidizing agent and solvent were 272 ml and must be heated to 100 °C. On the other hand, In the present invention for the oxidation of same amount of 2-methylnaphthalene with 100 % yield and selectivity, total amount of oxidizing agent and solvent are 225 ml and heated to 75 °C (Table 9).

**Table 9: Amount of reactants and temperature in W02002079133 and in the present invention.**

| | Amount of 30% aqueous hydrogen peroxide added (ml) | Amount of acetic acid added (ml) | Reaction temperature (°C) | **Total amount of oxidizing agent and solvent (ml)** | Yield and selectivity of 2-methyl-1,4-naphthoquinone (%) |
|---|---|---|---|---|---|
| W02002 079133 | 72 | 200 | 100 | **272** | 100 |
| Present invention | 144 | 81 | 75 | **225** | 100 |

In brief, the present invention does not include any metal oxidizing agents, transition metal catalysts or mineral acid for the oxidation of 2-methylnaphthalene to produce 2-methyl-1,4-naphthoquinone. The present invention provides an economical and ecofriendly process for the 100 % yield and regloselectivity of target product 2-methyl-1,4-naphthoquinone by using different molar ratios of 2-methylnaphthalene to hydrogen peroxide and acetic acid which is glacial acetic acid (conc. 17,4) and reaction time than the prior art processes.

## Claims

1. A process for producing 2-methyl-1,4-naphthoquinone by oxidation of 2-methylnaphthalene, including the steps of:
a) Oxidizing 2-methylnaphthalene with fresh 30 % aqueous solution of hydrogen peroxide where the molar ratios of 2-methylnaphthalene to hydrogen peroxide in the range of 1:15 to 1:30; and
b) Oxidizing 2-methylnaphthalene in the presence of 17.4 N glacial acetic acid, where the molar ratios of 2-methylnaphthalene to glacial acetic acid in the range of 1:20 to 1:30.

2. A process for producing 2-methyl-1,4-naphthoquinone according to claim 1, **characterized In that** the reaction time is in the period of 4-6 hours.

3. A process for producing 2-methyl-1,4-naphthoquinone according to claim 1, **characterized in that** the reaction temperature is in the range of 70-90°C.

4. A process for producing 2-methyl-1,4-naphthoquinone according to claim 1, **characterized in that** the oxidation reaction is carried out without any mineral acid or transition metal catalyst.

## Patentansprüche

1. Verfahren zur Herstellung 2-Methyl-1,4-Naphthochinon durch Oxidation von 2-Methylnaphthalin, umfassend die folgenden Schritte:
a) Oxidation 2-Methylnaphthalin mit frische 30% wässrigen Lösung von Wasserstoffperoxid, wobei Molverhältnisse von 2- Methylnaphthalin zu Wasserstoffperoxid im Bereich von 1:15 bis 1:30: und
b) Oxidation 2-Methylnaphthalin In Gegenwart von 17.4 N Eisessig, wobei Molverhältnisse von 2- Methylnaphthalin zu Eisessig im Bereich von 1:20 bis 1:30.

2. Verfahren zur Herstellung 2-Methyl-1,4-Naphthochinon nach Anspruch 1, **dadurch gekennzeichnet, dass** die Reaktionszeit im Zeitraum von 4-6 Stunden beträgt.

3. Verfahren zur Herstellung 2-Methyl-1,4-Naphthochinon nach Anspruch 1, **dadurch gekennzeichnet, dass** die Reaktionstemperatur im Bereich von 70-90°C beträgt.

4. Vertahtren zur Herstellung 2-Methyl-1,4-Naphthochinon nach Anspruch 1, **dadurch gekennzeichnet, dass** die Oxidationsreaktion ohne Mineralsäure oder Übergangsmetallkatalysators durchgeführt ist.

## Revendications

1. Procédé pour la production de 2-méthyl-1,4 naphtoquinone par oxydation de 2-méthylnaphtalène, comprenant les étapes consistant :
a) Oxydant 2- méthylnaphtalène avec une solution fraîche aqueuse de 30% de hydrogène peroxyde où rapport molaire de 2- méthylnaphtalène et hydrogène peroxyde est dans la plage de 1 :15 à 1 :30 et
b) Oxydant 2- méthylnaphtalène dan le 17.4 N acide acétique glacial, où rapport molaire de 2- méthylnaphtalène et acide acétique glacial est dans le plage de 1 :20 à 1 :30.

2. Procédé pour la production de 2-méthyl-1,4 naphtoquinone selon la revendication 1, caractérisé que la durée de réaction es dans la plage de 4-6 heures.

3. Procédé pour la production de 2-méthyl-1,4 naphtoquinone selon la revendication 1 caractérisé que la température de réaction est dans la plage de 70-90°C

4. Procédé pour la production de 2-méthyl-1,4 naphtoquinone selon la revendication 1 caractérisé que la réaction d'oxydation est effectué sans tout acide minéral ou la transition de métal catalyseur.
